# EUROPEAN PATENT APPLICATION

(11) **EP 4 477 219 A1**
(43) Date of publication of application: **18.12.2024**
(21) Application number: 23887661.9
(22) Date of filing: 19.09.2023
(51) Int. Cl.: A61K 31/433, A61K 38/12, A61P 31/04

(54) **USE OF SU3327 IN PREPARING DRUG FOR ENHANCING ANTI-BACTERIAL-INFECTION EFFICACY OF POLYMYXIN**

(30) Priority: 08.11.2022 CN 202211394623; 10.02.2023 CN 202310092613
(71) Applicant: Xiamen Hanlixin Pharmaceutical Co., Ltd., Xiamen, Fujian 361000 (CN); China Agricultural University, Haidian District, Beijing 100000 (CN)
(72) Inventor: SHEN, Jianzhong, Beijing 100000 (CN); DAI, Chongshan, Beijing 100000 (CN); CHEN, Hongliang, Xiamen, Fujian 361000 (CN); WU, Congming, Beijing 100000 (CN); SHEN, Zhangqi, Beijing 100000 (CN); WANG, Yang, Beijing 100000 (CN)
(74) Representative: ABG Intellectual Property Law, S.L.
(86) International application number: PCT/CN2023/119768
(87) International publication number: WO 2024/098967

(57) **Abstract**

The present invention provides use of a C-JUN N-terminal kinase inhibitor, SU3327, in the preparation of a medicament for enhancing the efficacy of polymyxin against bacterial infection, and use of a composition of SU3327 and polymyxin in the preparation of a medicament for an enhanced efficacy against bacterial infection, wherein the polymyxin is preferably Polymyxin E. The invention not only shows an enhanced antibacterial activity of polymyxin by SU3327 in synergy through minimum inhibitory concentration tests in checkerboard assays and *in vitro* bacterial growth curves, but also confirms the ability of SU3327 to effectively enhance polymyxin along with its effectiveness *in vivo* in the experiments with a model of mice infected with drug resistant bacteria at the animal level. The invention provides a new use of SU3327 in enhancing antibacterial activities of polymyxin antibiotics, which can resolve the technical problems such as clinical resistance to polymyxin, low clinical therapeutic index of polymyxin etc.

## Description

### Technical Field

The invention relates to the field of medicine, and in particular, to the antibacterial field, particularly to a method for enhancing the antibacterial activity of polymyxin by using a C-JUN N-terminal kinase inhibitor, SU3327.

### Background

Polymyxin, an antibacterial polypeptide found in the culture solution of *Bacillus polymyxa,* is classified into five types, i.e., A, B, C, D and E. The antibacterial spectrum is similar with each other and has a broad range, and is found to have a strong effect, especially, on Gram-negative bacteria, such as *Escherichia coli, Bacillus pneumonia,* and *Pseudomonas aeruginosa.* Polymyxin E (also known as colistin), CAS No.: 1066-17-7, English Name: Colistin, Trade Names: Kangdisu, Kelisiding and Nianjunsu. They are mainly clinically used in the form of sulfates or methanesulfonates, i.e., Polymyxin E sulfate, and Polymyxin E methanesulfonate. Polymyxin E finds use in treating enteritis caused by *Escherichia coli,* pneumonia caused by *Klebsiella pneumoniae,* and other drug resistant strains, as well as external use in topical infection with *Pseudomonas aeruginosa* resulting from burns and trauma, and infection with sensitive bacteria in sites such as ears, eyes etc.

Over the past 10 years, infections caused by multidrug resistant Gram-negative bacteria have posed a serious threat to animal and human health. At present, polymyxin antimicrobial drugs are considered to be the last option as a first-line therapy for clinical treatment of infection with multidrug resistant Gram-negative bacteria (mainly including Carbapenem resistant *Escherichia coli,* multidrug resistant *Klebsiella pneumonia* etc.). However, the presence of polymyxin resistant genes (MCR) results in a significant reduction in clinical therapeutic output of polymyxin, posing a severe threat to the lifetime of this essential antimicrobial drug. Therefore, development of effective colistin synergists, and co-administration based on colistin have become an important therapeutic strategy in the clinical treatment of these life-threatening multidrug resistant Gram-negative bacteria.

In order to cope with the "crisis of bacterial resistance" in clinical practice, combination therapy has become the most important medication strategy for the clinical use of polymyxin to treat MDR-GNR. Various combination therapies with polymyxin have been disclosed in the literature. The minimum inhibitory concentrations (MIC) of 17 different antimicrobial drugs against 135 strains of Carbapenem resistant *Klebsiella pneumoniae* (CRKP) were determined to evaluate the sensitivities of these CRKP strains to the drugs. The synergistic antibacterial activity of two combination therapy regimens, colistin based combination with biapenem, and ceftazidime-avibactam, on CRKP was evaluated to provide a theoretical basis for clinical medication selection (see "Study on the in vitro Antibacterial Activity of Colistin Combined with Other Antibacterial Drugs against Carbapenem Resistant Klebsiella pneumoniae", Bai Yan et al., Beijing Annual Pharmaceutical Conference 2016, published in 2016). A pharmacodynamic model was established to describe the *in vitro* bactericidal effect of Polymyxin E (colistin E) combined with other antibacterial drugs on pan-drug resistant *Acinetobacter baumannii* (XDR-AB), and the established pharmacodynamic model had well described the characteristics of the bactericidal effect of Polymyxin E on XDR-AB, which provides an exemplification for modeling pharmacokinetic/pharmacodynamic data from combination of antibacterial drugs *in vitro* (see "Pharmacodynamic Modeling for in vitro Evaluation of Colistin E Based Combination Treatments for Acinetobacter baumannii Infection", Fu Wenting et al., Anhui Medical and Pharmaceutical Journal, 2012).

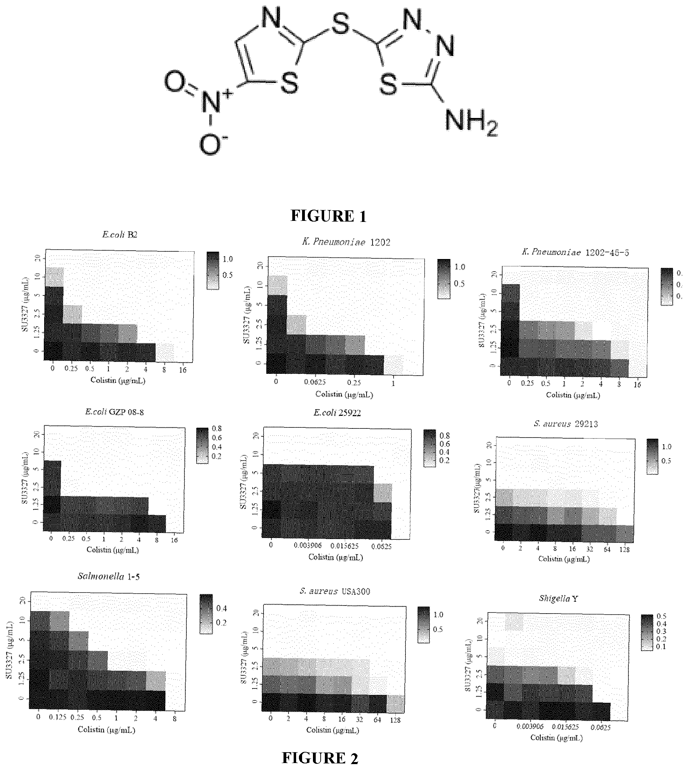

SU3327 (also known as halicin, or Hailixin, CAS No.: 40045-50-9) is an N-terminal kinase inhibitor of C-JUN, whose structure is shown in Fig. 1. SU3327 is an effective, selective, and substrate-competitive JNK inhibitor with an IC₅₀ of 0.7µM. SU3327 also inhibited protein interaction between JNK and JIP with an IC₅₀ value of 239nM. SU3327 has low activity for p38α and Akt kinases.

At present, despite the disclosure in the prior art of polymyxin in combination with other antibacterial drugs, most of them have poor druggability. The present invention not only provides experiments with a combination of SU3327 and polymyxin at a cell level for a synergistic, enhanced effect, but also provides experiments at the animal level for a good, confirmed synergistic bactericidal effect. To date, no study has been reported on use of SU3327 as a synergist of polymyxin in enhancing the antibacterial activity of polymyxin.

### Summary

In order to solve the technical problem, i.e., treatment of multidrug resistant Gram-negative bacteria, the present invention provides a method for enhancing the antibacterial activity of polymyxin, by using in particular a C-JUN N-terminal kinase inhibitor, SU3327, in combination with polymyxin, wherein polymyxin is preferably Polymyxin E; they are more than a sum of their functions, but achieves a synergistic, enhanced antibacterial effect. The present invention also discloses a ratio of SU3327 to Polymyxin E as an antibacterial combination, providing a new therapeutic strategy for clinical treatment of bacterial infection, in particular, multidrug resistant Gram-negative bacterial infection, and specifically, infection with bacteria having polymyxin resistant genes (MCR), for example.

One aspect of the invention provides use of a C-JUN N-terminal kinase inhibitor, SU3327, in the preparation of a medicament for enhancing the efficacy of polymyxin against bacterial infection.

Another aspect of the invention provides use of a composition of a C-JUN N-terminal kinase inhibitor SU3327 and polymyxin in the preparation of a medicament for an enhanced efficacy against bacterial infection.

Further, the polymyxin is Polymyxin E (i.e., colistin), or Polymyxin B.

Further, the bacteria are bacteria with polymyxin resistant genes.

Further, the bacteria are Gram-negative bacteria.

Further, the Gram-negative bacteria are multidrug resistant Gram-negative bacteria.

Further, the bacteria are one or more of *Escherichia coli, Klebsiella pneumoniae, Salmonella, Shigella* and *Staphylococcus aureus;* preferably, the one or more of *Escherichia coli, Klebsiella pneumoniae, Salmonella, Shigella* and *Staphylococcus aureus* are those with polymyxin resistant genes or multidrug resistance.

Further, the bacteria are *Klebsiella pneumoniae;* preferably, the bacteria are *Klebsiella pneumoniae* with polymyxin resistant genes or multiple drug resistance.

Further, a mass ratio of SU3327 to polymyxin in the composition of SU3327 and polymyxin is: (2.5-10): 1.

Further, the dosage form of the composition of SU3327 and polymyxin is one of tablets, capsules, sustained release tablets, controlled release tablets, oral solutions, syrups, dosage forms for injection, dripping pills, and dosage forms of lyophilized powders for injection.

Further, the final therapeutic dose of the Polymyxin E and the SU3327 is 10mg/kg body weight.

The new use of a C-JUN N-terminal kinase inhibitor, SU3327, provided by the invention - a method for enhancing the antibacterial activity of polymyxin - has excellent technical effects as follows:
(1) The present invention demonstrates an enhanced antibacterial activity of polymyxin by SU3327 in synergy through minimum inhibitory concentration tests in checkerboard assays and *in vitro* bacterial growth curves.
(2) Distinct from the existing combinations of polymyxin with antibiotics, the invention provides experiments with a model of mice infected with drug resistant bacteria, confirming at a animal level the ability of SU3327 to effectively enhance polymyxin along with its effectiveness *in vivo* - a more convincing evidence for the subsequent clinical application.
(3) The invention throws light upon the ability of SU3327 to restore the sensitivity of bacteria to polymyxin, and further evaluates the effectiveness of combination use of the two *in vivo* and *in vitro,* facilitating the development of a new type of antibiotic synergists and mitigating the increasingly harmful bacterial resistance to drugs.
(4) The invention provides a new use of SU3327 in enhancing antibacterial activities of polymyxin antibiotics, which can resolve the technical problems such as clinical resistance to polymyxin, low clinical therapeutic index of polymyxin etc.

### Brief Description of Drawings

Fig. 1 is a diagram depicting the chemical structure of SU3327, according to the invention;
Fig. 2 shows the test results of the antibacterial activities against strains of SU3327 alone, Polymyxin E alone, and in combination in checkerboard assays, according to the invention;
Figs. 3A-3B are graphs showing the results of *in vitro* bacterial growth curves for *K.Pneumoniae* 1202 and *K.Pneumoniae* 1202-45-5 when given SU3327 alone, Polymyxin E alone, and in combination, wherein Fig. 3A is a graph showing the results of *in vitro* bacterial growth curves for the strain *K.Pneumoniae* 1202 when given SU3327 alone, Polymyxin E alone, and in combination, and Fig. 3B is a graph showing the results of *in vitro* bacterial growth curves for the strain *K.Pneumoniae* 1202-45-5 when given SU3327 alone, Polymyxin E alone, and in combination, according to the invention;
Fig. 4 is a graph showing the results of the bacteria loadings in the thighs of the mice treated with SU3327 alone, Polymyxin E alone, and in combination, for *K.Pneumoniae* 1202-45-5, according to the invention.

### Detailed Description

Medicaments used in the examples of the present invention: SU3327, purchased from MedChemexpress CO., Ltd, USA, purity ≥ 99%; Polymyxin E sulfate, purchased from Hebei Sacred Snow Dacheng Tangshan Pharmaceutical Co., Ltd., titer ≥ 23,000U/mg Polymyxin E. A certain amount of Polymyxin E sulfate was weighed to formulate an aqueous solution with a stock solution concentration of 16mg/mL. SU3327 was formulated with DMSO as a stock solution with a stock solution concentration of 40mg/mL. All stock solutions were prepared and kept in a -20°C refrigerator.

### Example 1 Evaluation of Synergistic Antibacterial Activity of combination adminstration of SU3327 and Polymyxin

### 1.1 Test Strains

The strains used in the tests, the strain *Escherichia coli* ATCC 25922 (*E.coli* 25922) and the strain *Staphylococcus aureus* ATCC 29213 (*S.aureus* 29213), were purchased from China Veterinary Culture Collection Center. Methicillin Resistant *Staphylococcus Aureus* USA300 (ATCC BAA-1717) was purchased from American Type Culture Collection. The strains used in the other experiments, the strains *Escherichia coli* B2 (*E.coli* B2), *Escherichia coli* GZP08-8 (*E.coli* GZP08-8), *Klebsiella pneumoniae* 1202 (*K.Pneumoniae* 1202), *Klebsiella pneumoniae* 1202-45-5 (*K.Pneumoniae* 1202-45-5), *Salmonella* 1-5 (*Salmonella* 1-5), and *Shigella* Y (*Shigella* Y) were all from National Center for Veterinary Drug Safety Evaluation of China Agricultural University, with *Escherichia coli* B2 (*E.coli* B2), *Escherichia coli* GZP08-8 (*E.coli* GZP08-8), *Klebsiella pneumoniae* 1202-45-5 (*K.Pneumoniae* 1202-45-5), and *Salmonella* 1-5 (*Salmonella* 1-5) as polymyxin resistant strains containing MCR-1. *Klebsiella pneumoniae* 1202 (*K.Pneumoniae* 1202) and *Shigella* Y (*Shigella* Y) were strains clinically sensitive to colistin.

### 1.2 Test Methods

The MIC of SU3327 administered alone was detected with dilution by factors. Subsequently, tests were performed with SU3327 alone, Polymyxin E (Colistin) alone, and in combination, respectively, in sterile, 96-well microplates according to checkerboard assays for the joint inhibitory activities against the above-mentioned strains, and further Fractional Inhibitory Concentration Index (FICI) was calculated. FICI of SU3327 and Polymyxin E = MIC (Polymyxin E in combination) / MIC (Polymyxin E) + MIC (SU3327 in combination) / MIC (SU3327 alone). If FICI≤0.5, synergy was identified; if 0.5<FICI≤1, an additive effect was identified; and if 0.5<FICI<4, an irrelevant effect was identified.

### 1.3 Test Results

The test results of the antibacterial activities against strains of SU3327 alone, Polymyxin E alone, and in combination in checkerboard assays were shown in Fig. 2. The results were analyzed in detail as follows:
For *Escherichia coli* B2 (*E.coli* B2) containing MCR-1, the MICs of Polymyxin E and SU3327 were 8µg/mL and 20µg/mL, respectively. After use in combination, the MICs of Polymyxin E and SU3327 were lowered to 0.5µg/mL and 2.5µg/mL, respectively, and FICI was 0.1875, which was identified as synergy.

For a strain clinically sensitive to colistin, *Klebsiella pneumoniae* 1202 (*K.Pneumoniae* 1202), the MICs of Polymyxin E and SU3327 were 1µg/mL and 20µg/mL, respectively. After use in combination, the MICs of Polymyxin E and SU3327 were lowered to 0.0625µg/mL and 2.5µg/mL, respectively, and FICI was 0.1875, which was identified as synergy.

For a strain sensitive to colistin, *Klebsiella pneumoniae* 1202-45-5 (*K.Pneumoniae* 1202-45-5), the MICs of Polymyxin E and SU3327 were 16µg/mL and 20µg/mL, respectively. After use in combination, the MICs of Polymyxin E and SU3327 were lowered to 0.25µg/mL and 5µg/mL, respectively, and FICI was 0.266, which was identified as synergy.

For a strain sensitive to colistin, *Escherichia coli* GZP08-8 (*E.coli* GZP08-8), the MICs of Polymyxin E and SU3327 were 16µg/mL and 10µg/mL, respectively. After use in combination, the MICs of Polymyxin E and SU3327 were lowered to 0.25µg/mL and 2.5µg/mL, respectively, and FICI was 0.266, which was identified as synergy.

For a standard *Escherichia coli* strain ATCC 25922 (*E. coli* 25922), the MICs of Polymyxin E and SU3327 were 0.125µg/mL and 10µg/mL, respectively. After use in combination, the MICs of Polymyxin E and SU3327 were lowered to 0.0625µg/mL and 5µg/mL, respectively, and FICI was 1, which was identified as an additive effect.

For a standard *Staphylococcus aureus* strain ATCC 29213 (*S. aureus* 29213), the MICs of Polymyxin E and SU3327 were more than 128µg/mL and 5µg/mL, respectively. After use in combination, the MICs of Polymyxin E and SU3327 were lowered to 64µg/mL and 2.5µg/mL, respectively, and FICI was 0.75, which was identified as an additive effect.

For a colistin resistant strain, *Salmonella* 1-5 (*Salmonella* 1-5), the MICs of Polymyxin E and SU3327 were more than 128µg/mL and 20µg/mL, respectively. After use in combination, the MICs of Polymyxin E and SU3327 were lowered to 1µg/mL and 2.5µg/mL, respectively, and FICI was 0.25, which was identified as synergy.

For a methicillin resistant strain, *Staphylococcus Aureus* USA300 (*S.aureus* USA300), the MICs of Polymyxin E and SU3327 were more than 128µg/mL and 5µg/mL, respectively. After use in combination, the MICs of Polymyxin E and SU3327 were lowered to 32µg/mL and 1.25µg/mL, respectively, and FICI was 0.25, which was identified as synergy.

For a colistin sensitive strain, *Shigella* Y (*Shigella* Y), the MICs of Polymyxin E and SU3327 were 0.125µg/mL and 5µg/mL, respectively. After use in combination, the MICs of Polymyxin E and SU3327 were lowered to 0.032µg/mL and 2.5µg/mL, respectively, and FICI was 0.75, which was identified as an additive effect.

### Example 2 Synergistic Bactericidal Curves for Use of Polymyxin E in Combination with SU3327

### 2.1 Formulation of MHA Medium

6.0g powdered beef extract, 1.5g soluble starch, 17.5g acid hydrolysate of casein, and 17.0g agar were weighed, to which 900mL distilled water was added, with the pH adjusted to 7.3, and the volume to 1000mL. The mixture was subject to autoclave at 121°C for 15 minutes, and then cooled to 50°C, poured into a sterile plate, and dried for later use.

### 2.2 Test Methods

Following culturing of *K.Pneumoniae* 1202 and *K.Pneumoniae* 1202-45-5 in BHI broth for 6-8 hours, the bacteria were diluted to a concentration of 10⁶ colony count using a turbidimeter. For *K.Pneumoniae* 1202, drug treatment concentrations were set as follows: Polymyxin E (0.25µg/mL), SU3327 (10µg/mL), SU3327 (10µg/mL) + Polymyxin E (0.25µ g/mL); the bacteria in control group were treated with a solvent, 0.1% DMSO. For *K.Pneumoniae* 1202-45-5, drug treatment concentrations were set as follows: Polymyxin E (1µg/mL), SU3327 (20µg/mL), SU3327 (20µg/mL) + Polymyxin E (1µg/mL). 100µL of a bacterial solution was applied on an agar plate at 1h, 3h, 6h, 12h and 24h post drug treatment, respectively, and cultured for at least 16h for a colony count.

### 2.3 Test Results

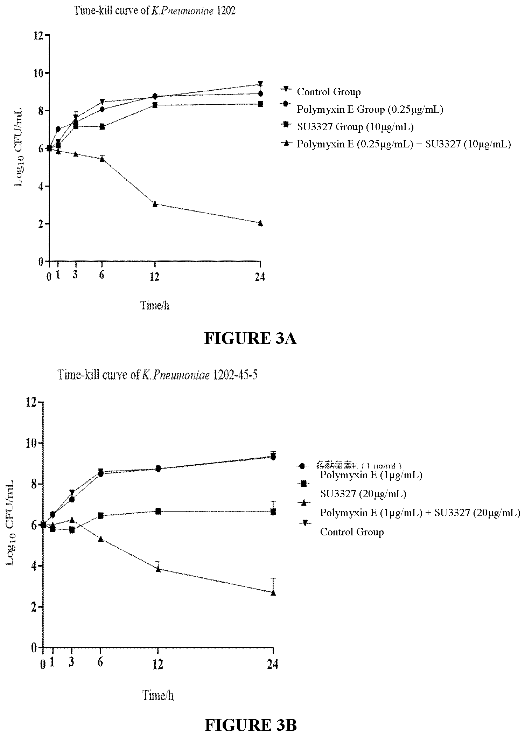

The graphs showing the results of *in vitro* bacterial growth curves for *K.Pneumoniae* 1202 and *K.Pneumoniae* 1202-45-5 when given SU3327 alone, Polymyxin E alone, and in combination, can be seen in Figures 3A-3B, wherein Fig. 3A is a graph showing the results of *in vitro* bacterial growth curves for the strain *K.Pneumoniae* 1202 when given SU3327 alone, Polymyxin E alone, and in combination, and Fig. 3B is a graph showing the results of *in vitro* bacterial growth curves for the strain *K.Pneumoniae* 1202-45-5 when given SU3327 alone, Polymyxin E alone, and in combination. The results were analyzed in detail as follows:
The results from *K.Pneumoniae* 1202 showed that a combined treatment group of SU3327 (10µg/mL) and Polymyxin E (0.25µg/mL) exhibited a significantly higher bactericidal effect than either SU3327 or Polymyxin E as a single treatment; following continuous treatment for 24h, the bacterial colony counts were 9.4 Log10 CFU/mL in the control treatment group, 8.9 Log10 CFU/mL in the Polymyxin E treatment group, 8.35 Log10 CFU/mL in the SU3327 treatment group, and 2.05 Log10 CFU/mL in the combined treatment group of Polymyxin E and SU3327; it suggested that the bactericidal effect was significantly improved after treatment with Polymyxin E in combination with SU3327.

The results from *K.Pneumoniae* 1202-45-5 showed that a combined treatment group of SU3327 (20µg/mL) and Polymyxin E (1µg/mL) exhibited a significantly higher bactericidal effect than either SU3327 or Polymyxin E as a single treatment; following continuous treatment for 24h, the bacterial colony counts were 9.35 Log10 CFU/mL in the control treatment group, 9.3 Log10 CFU/mL in the Polymyxin E treatment group, 6.65 Log10 CFU/mL in the SU3327 treatment group, and 2.7 Log10 CFU/mL in the combined treatment group of Polymyxin E and SU3327; it suggested that the bactericidal effect was significantly improved after treatment with Polymyxin E in combination with SU3327.

### Experimental Example 3 Effects of Use of Polymyxin E alone, SU3327 alone, and in Combination on the Treatment of a Model of BALB/c mice Infected with Drug Resistant Bacteria

### 3.1 Animal Grouping and Treating

In order to avoid the influence of mice's own immunity on the experiments, all mice were administered in advance with cyclophosphamide twice, four days and one day before infection, respectively, 100mg/kg intraperitoneal injection each time, resulting in neutropenia and immune deficiency in the mice. 24 female BALB/c mice (weighing about 20g) aged 6-8 weeks old were prepared. *K.Pneumoniae* 1202-45-5 in logarithmic phase was re-suspended with a PBS buffer to prepare a suspension at the concentration of 1×10⁷CFU/mL; in the mice, the total amount of bacteria injected into the thigh of each mouse was 1×10⁶CFUs in the end. Then they were randomly divided into model control group (solvent treatment group), Polymyxin E treatment group (10mg/kg body weight), SU3327 treatment group (10mg/kg body weight), and combined treatment group of SU3327 (10mg/kg body weight) + Polymyxin E (10mg/kg body weight) (6 mice in each group). The treatments are detailed as follows:
Solvent control treatment group: 0.1mL of the suspension of *K.Pneumoniae* 1202-45-5 was intramuscularly injected into a mouse in the thigh on the right side of its abdominal cavity; one hour later, the mouse was intraperitoneally injected with 200µL of a PBS buffer.
Polymyxin E treatment group: 0.1mL of the suspension of *K.Pneumoniae* 1202-45-5 was intramuscularly injected into a mouse in the thigh on the right side of its abdominal cavity; one hour later, the mouse was intraperitoneally injected with 200µL of a solution of drug, Polymyxin E (1mg/mL); the final dose was 10mg/kg body weight;
SU3327 treatment group: 0.1mL of the suspension of *K.Pneumoniae* 1202-45-5 was intramuscularly injected into a mouse in the thigh on the right side of its abdominal cavity; one hour later, the mouse was intraperitoneally injected with 200µL of a solution of drug, SU3327 (1mg/mL); the final dose was 10mg/kg body weight;
Combined treatment group of SU3327 (10mg/kg) + Polymyxin E (10mg/kg): 0.1mL of the suspension of *Escherichia coli* was intramuscularly injected into a mouse in the thigh on the right side of its abdominal cavity; one hour later, the mouse was intraperitoneally injected with a mixed solution of drugs, SU3327 (1mg/mL) and 200µL of Polymyxin E (1mg/mL). The final dose was 10mg/kg body weight for each of SU3327 and Polymyxin E. Treatment was conducted twice with an interval of 8 hours between treatments. At 24 hour post infection, all the mice were euthanized, and the muscles of the right thighs of the mice were taken and placed in 3mL of a PBS solution, and then broken up by a low-temperature tissue disruptor. Finally, 100µL of the breakup liquid was smeared on a plate for a colony count.

### 3.2 Test Results

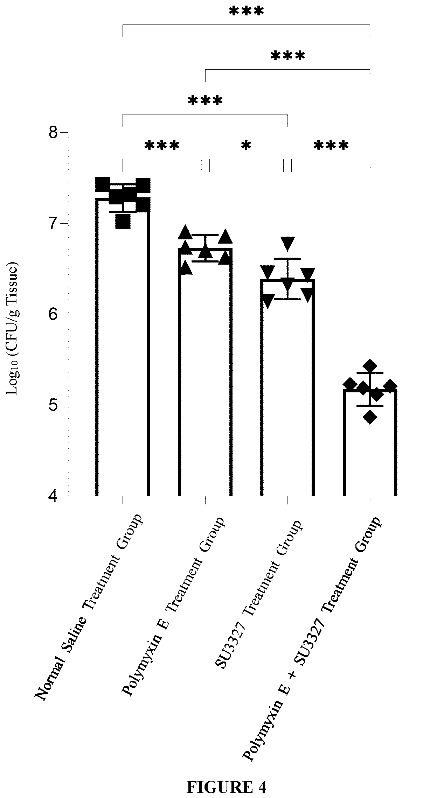

A graph showing the results of the bacteria loadings in the thighs of the mice treated with SU3327 alone, Polymyxin E alone, and in combination, against *K.Pneumoniae* 1202-45-5 is shown in Figure 4, and the results are as follows:
Compared with solvent control group, the bacteria loading in the thigh of the mice in the single treatment group of Polymyxin E was 6.82 Log10 CFU/mL, which was not significantly different from control group; compared with solvent control group, the bacteria loading in the thigh of the mice in the single treatment group of SU3327 was 6.55 Log10 CFU/mL, which was significantly different from control group; compared with solvent control group, the bacteria loading in the thigh of the mice in combined treatment group of Polymyxin E and SU3327 was 5.28 Log10 CFU/mL, which was significantly different from control group, and significantly different from either single treatment group of SU3327 or single treatment group of Polymyxin E, respectively. These results showed that SU3327 could effectively enhance Polymyxin E and its effectiveness *in vivo,* and provide a new therapy scheme for infectious diseases caused by drug resistant Gram-negative bacteria.

The above-mentioned examples in the disclosure are merely exemplifications for clearly illustrating the disclosure and are not intended to limit the embodiments of the disclosure. Other variations or modifications in various forms may be made by those skilled in the art to which they pertain from the foregoing description. All embodiments are not necessarily, or possibly exhaustive here. Any of modifications, equivalent substitutions, and improvements made within the spirit and principles of the disclosure are intended to be included within the scope of the claims of the disclosure.

## Claims

1. Use of a C-JUN N-terminal kinase inhibitor, SU3327, in the preparation of a medicament for enhancing the efficacy of polymyxin against bacterial infection.

2. Use of a composition of a C-JUN N-terminal kinase inhibitor, SU3327 and polymyxin in the preparation of a medicament with an enhanced efficacy against bacterial infection.

3. The use of claim 1 or 2, **characterized in that** the polymyxin is Polymyxin E or Polymyxin B; preferably, the polymyxin is Polymyxin E.

4. The use of any of claims 1-3, **characterized in that** the bacteria are bacteria with polymyxin resistant genes.

5. The use of any of claims 1-3, **characterized in that** the bacteria are Gram-negative bacteria.

6. The use of claim 5, **characterized in that** the Gram-negative bacteria are multidrug resistant Gram-negative bacteria.

7. The use of any of claims 1-3, **characterized in that** the bacteria are one or more of *Escherichia coli, Klebsiella pneumoniae, Salmonella, Shigella* and *Staphylococcus aureus;* preferably, the one or more of *Escherichia coli, Klebsiella pneumoniae, Salmonella, Shigella* and *Staphylococcus aureus* are those with polymyxin resistant genes or multidrug resistance.

8. The use of any of claims 1-3, **characterized in that** the bacteria are *Klebsiella pneumoniae;* preferably, the bacteria are *Klebsiella pneumoniae* with polymyxin resistant genes or multiple drug resistance.

9. The use of any of claims 2-8, **characterized in that** a mass ratio of SU3327 to polymyxin in the composition of SU3327 and polymyxin is (2.5-10):1.

10. The use of any of claims 2-8, **characterized in that** the dosage form of the composition of SU3327 and polymyxin is one of tablets, capsules, sustained release tablets, controlled release tablets, oral solutions, syrups, dosage forms for injection, dripping pills, and dosage forms of lyophilized powders for injection.

11. The use of any of claims 2-8, **characterized in that** the final therapeutic dose of the Polymyxin E and the SU3327 is 10mg/kg body weight.
